# EUROPEAN PATENT SPECIFICATION

(11) **EP 2 325 640 B1**
(45) Date of publication and mention of the grant of the patent: **15.03.2017**
(21) Application number: 09808308.2
(22) Date of filing: 21.08.2009
(51) Int. Cl.: G01N 33/558

(54) **TEST APPARATUS FOR MEMBRANE ASSAY EQUIPPED WITH REFERENCE DISPLAY SECTION**
TESTGERÄT FÜR MEMBRANASSAY MIT REFERENZANZEIGEBEREICH
APPAREIL D' ANALYSE POUR ESSAI SUR MEMBRANE ÉQUIPÉ D'UNE SECTION D'AFFICHAGE DE RÉFÉRENCE

(30) Priority: 22.08.2008 JP 2008214226
(43) Date of publication of application: 25.05.2011
(73) Proprietor: DENKA SEIKEN Co., Ltd., Tokyo 103-8338 (JP)
(72) Inventor: MAEGAWA Tsuneo, Gosen-shi Niigata 959-1695 (JP)
(74) Representative: Hoffmann Eitle
(86) International application number: PCT/JP2009/064623
(87) International publication number: WO 2010/021372

(56) References cited:
- WO-A1-97/09620
- WO-A1-03/058246
- WO-A1-2006/034385
- WO-A2-2005/098439
- JP-T- 2007 531 882
- US-A- 4 920 046
- US-A- 4 963 494
- US-A1- 2003 119 203
- US-A1- 2004 121 334

## Description

### Technical Field

The present invention relates to a test device for membrane assay using a specific binding reaction.

### Background Art

In recent years, test devices based on measurement principles of specific binding reactions, which actually do not require users to perform skilled and time-consuming procedures, have been increasingly used for tests in various fileds mainly related to clinical diagnosis, environmental analysis, and food sanitation control. There are various types of test devices based on measurement principles of specific binding reactions. Of these, lateral-flow-type and flow-through-type test devices for membrane assay have been widely spread.

In one configuration, a commonly-used lateral-flow-type test device is in the strip-like form and it comprises a porous membrane carrier inside which liquid can be transferred by capillary action. A detection display section on which a substance capable of specifically binding to a substance to be detected (capture reagent) has been immobilized is provided to one part of such membrane carrier. In addition, a substance capable of specifically binding to a substance to be detected which has been labeled with a labeling substance comprising an enzyme such as peroxidase or coloring particles such as gold colloidal particles (labeled reagent) is provided upstream of the membrane carrier of a strip in a manner such that it can be transferred inside the membrane carrier by capillary action. For instance, when a specific binding reaction is an antigen-antibody reaction in which an antigen is a substance to be detected, an antibody capable of specifically binding to a substance to be detected is used as a capture reagent and a labeled antibody capable of specifically binding to a substance to be detected is used as a labeled reagent. In such case, when a liquid sample is supplied to a site located upstream of a strip where a labeled reagent has been provided, the liquid sample comes into contact with the labeled reagent, resulting in dissolution or dispersion of the labeled reagent. If a substance to be detected is present in the liquid sample, the dissolved or dispersed labeled reagent binds to the substance such that a "labeled reagent-substance to be detected" complex is formed. The "labeled reagent-substance to be detected" complex is further transferred downstream of the strip and arrives at a detection display section. In the detection display section, the "labeled reagent-substance to be detected" complex is captured by a capture reagent in the detection display section such that a "labeled reagent-substance to be detected-capture reagent" sandwich complex is formed. Then, the labeling substance of the labeled reagent constituting such sandwich complex in the detection display section is visually observed. Detection is carried out by an arbitrary manner such as color development for determination of the presence or absence of a substance to be detected (see Patent Documents 1 to 4).

In one configuration, a commonly used flow-through-type test device comprises a porous membrane carrier through which liquid can pass. A detection display section on which a substance capable of specifically binding to a substance to be detected (capture reagent) has been immobilized is provided to one portion of the upper surface of such membrane carrier and a water-absorbing means is provided onto the lower surface of the membrane carrier. For instance, when a specific binding reaction is an antigen-antibody reaction in which an antigen is a substance to be detected, an antibody serving as a capture reagent capable of specifically binding to a substance to be detected has been immobilized to one part of the upper surface of the membrane carrier. In such case, when a liquid sample is supplied to the upper surface of a membrane carrier, the liquid sample passes through the membrane carrier so as to be absrobed by a water-absorbing means. If a substance to be detected is present in a sample, the substance to be detected is captured by the capture reagent immobilized in the detection display section provided to the upper surface of the membrane carrier such that a "substance to be detected-capture reagent" complex is formed in the detection display section. Subsequently, an antibody capable of specifically binding to a substance to be detected which has been labeled with a labeling substance comprising an enzyme such as peroxidase or coloring particles such as metallic colloidal particles (labeled reagent) is supplied to the upper surface of the membrane carrier. Thus, the antibody is allowed to bind to the substance to be detected that has been captured in the detection display section. Accordingly, a "labeled reagent-substance to be detected-capture reagent" sandwich complex is formed in the detection display section. Then, the labeling substance of the labeled reagent constituting such sandwich complex in the detection display section is visually observed. Detection is carried out by an arbitrary manner such as color development for determination of the presence or absence of a substance to be detected (see Patent Documents 5 to 7).

A reference display section that displays reference test results indicating proper test completion to an operator is provided to the above test devices. When a substance to be detected is not present in a sample, such a reference display section indicates the passage of a sample and a labeled reagent through a membrane carrier even if a labeling substance is not detected in a detection display section. For instance, in a case in which a substance to be detected is not detected in a detection display section, if the passage of a sample and a labeled reagent through a membrane carrier can be confirmed in a reference display section, proper test completion is verified. On the other hand, if the passage of a sample and a labeled reagent through a membrane carrier or a strip cannot be confirmed in a reference display section, it can be considered that there is a failure related to a sample or a labeled reagent due to some reason. This indicates improper test completion. In such case, the obtained test results are considered invalid. Then, the sample is retested.

In many cases, a test device comprising a reference display section that allows an antigen-antibody reaction to a labeled reagent has been used. For example, a substance identical to a substance to be detected or a substance having immunological specific reactivity to a labeled reagent that is identical to that of a substance to be detected is immobilized in a reference display section of a membrane carrier (see Patent Document 8). However, a reference test using an antigen-antibody reaction is likely to be influenced by the amount of an antigen contained in a sample, types of foreign substances, pH, and other factors. This results in poor display sensitivity and unclear display in a reference display section in some cases. Therefore, a test device provided with a reference display section showing improved stability has been awaited.

There is an invention of a test device in which a color former or indicator is used in a reference display section using a reaction other than an antigen-antibody reaction to a labeled reagent. For instance, there is a lateral-flow-type test device provided with a reference display section, wherein the reference display section is configured such that it indicates the completion of a reaction by allowing filter paper to absorb a color former comprising bromocresol green, 2,5-dinitrophenol, or bromphenol blue and drying the filter paper (see Patent Document 9). In this case, color development takes place when the color former is moistened with water, indicating the completion of a reaction. However, in one case of this method, normal color development of a color former does not take place depending on the pH of a sample. In another case, even if normal color development takes place, a color former flows out with a liquid sample when the sample passes through the reference display section. Therefore, a reference test cannot be stably carried out. Further, there is a lateral-flow-type test device in which a reference test is carried out such that the completion of a reaction is indicated by allowing filter paper to directly absorb a dye used as an indicater containing benzopurpurine 4B or congo red for staining (see Patent Document 10). However, in this method, filter paper is used for retaining an indicator and thus it is necessary to incorporate filter paper into a test device. Thus, the number of test device component parts is increased, resulting in complicated assembly. In addition, there is an invention that has been made in view of the problematic points of the inventions disclosed in Patent Documents 9 and 10. The invention relates to a lateral-flow-type test device in which a reference test is carried out such that the completion of a reaction is indicated by allowing a membrane carrier to absorb an indicator comprising an acidic dye (containing at least one member selected from among eosin B, eosin Y, phloxine B, and bromphenol blue) dissolved in an acid-containing lower alcohol solution and drying the membrane carrier (see Patent Document 11). However, in cases of the above referece tests using a color former or indicator, the completion of a reaction can be confirmed when a liquid sample has passed through a membrane carrier in a proper manner. However, the passage of a labeled reagent through a membrane carrier in a proper manner cannot be verified.

Patent Document 12 describes a lateral flow device including a control zone which comprises polyethylenimine to immobilize probe-conjugates or probes that migrate to the control lines without analyte, thereby positioning them for generating a control signal indicating proper test completion.
Patent Document 1: JP Patent Publication (Kokoku) No. 7-78503 B (1995)
Patent Document 2: JP Patent Publication (Kokoku) No. 7-36017 B (1995)
Patent Document 3: JP Patent Publication (Kokoku) No. 6-27738 B (1994)
Patent Document 4: JP Patent Publication (Kokai) No. 1-244370 A (1989)
Patent Document 5: JP Patent Publication (Kokoku) No. 7-34016 B (1995)
Patent Document 6: JP Patent Publication (Kokoku) No. 7-113637 B (1995)
Patent Document 7: JP Patent Publication (Kokai) No. 3-118473 A (1991)
Patent Document 8: JP Patent Publication (Kokai) No. 2007-333426 A
Patent Document 9: JP Patent Publication (Kokai) No. 4-353764 A (1992)
Patent Document 10: JP Patent No. 2942087
Patent Document 11: JP Patent No. 3385377
Patent Document 12: US 2003/0119203

### Disclosure of the Invention

### Problem to be Solved by the Invention

An antigen-antibody reaction caused by a labeled antibody is used in reference display sections in many conventional test divices for membrane assay involving a specific binding reaction. An antigen-antibody reaction is likely to be influenced by the amount of an antigen contained in a sample, types of foreign substances, pH, and other factors. This results in poor display sensitivity and unclear display in reference display sections in some cases. In addition, a reference display section on which a color former or indicator is used cannot demonstrate the passage of a labeled antibody through a membrane carrier in a proper manner. The present invention has been made in view of conventional problems. An object of the present invention is to provide a test device provided with a reference display section that rapidly and clearly indicates proper test completion with improved accuracy and stability.

### Means for Solving Problem

As a result of intensive studies of a means for achieving the above obejct, the present inventors found that the use of a cationic substance in a reference display section for membrane assay involving a specific binding reaction allows rapid and clear display of reference test results, thereby achieving the implementation of a reference test with improved stability. This has led to the completion of the present invention.

Specifically, the present invention is in its broadest sense defined by the appended claims.

[1] A test device for membrane assay using a specific binding reaction of a substance to be detected with a capture reagent immobilized on a membrane carrier and a reagent labeled with a labeling substance, the reagent being an anionic substance or an ampholyte that is negatively charged under basic conditions, which comprises a reference display section for indicating proper test completion on which a cationic polymer for capturing a labeled reagent has been immobilized, wherein the cationic polymer is a polymer compound having a cation charge density of 0.4 meq/g to 21 meq/g, and an average molecular weight of 300 to 5,000,000, wherein the cationic polymer is polyallylamine, wherein the test device is a lateral-flow-type test device, and wherein the reference display section for indicating proper test completion is configured such that it is positioned downstream of a detection display section on which a capture reagent has been immobilized. [2] The test device according to [1], wherein the reference display section is configured by allowing the membrane carrier to absorb the cationic polymer and drying the membrane. [3] The test device according to [1] or [2], wherein the specific binding reaction is an antigen-antibody reaction. [4] A method for confirming the proper completion of a test for membrane assay using the test device according to any one of [1] to [3], the method using a specific binding reaction of a substance to be detected with the capture reagent immobilized on the membrane carrier and the reagent labeled with the labeling substance, whereby proper test completion can be confirmed when labeling of the labeled reagent is detected in the reference display section on the membrane by allowing the labeled reagent to bind to the cationic polymer immobilized in the reference display section.

This description includes part or all of the contents as disclosed in the description and/or drawings of Japanese Patent Application No. 2008-214226, which is a priority document of the present application.

### Effects of the Invention

According to the present invention, reference test results can be displayed more rapidly and clearly with substantially no influence due to the amount of an antigen contained in a sample, types of foreign substances, pH, and other factors. Therefore, a test device comprising a reference display section with improved accuracy and stability compared with cases involving conventional methods can be provided.

Further, an easy-to-produce test device comprising a reference display section for displaying results more rapidly and clearly than in cases involving conventional methods can be provided with the use of inexpensive polyallylamine as a cationic substance.

### Brief Description of the Drawings

Fig. 1 shows a top view and a cross-sectional view of a lateral-flow-type test kit in one embodiment of the present invention.
Fig. 2 shows a top view and a cross-sectional view of a flow-through-type test kit (Model 1).
Fig. 3 shows a top view and a cross-sectional view of a flow-through-type test kit (Model 2).

### Description of Reference Numerals

- 1:: Backing sheet
- 2:: Membrane carrier
- 3:: Impregnated member
- 4:: Sample supply member
- 5:: Water-absorbing member
- 6:: Detection display section
- 7:: Reference display section
- 8:: Plastic petri dish
- 9:: Water-absorbing pad
- 10:: Membrane carrier
- 11:: Detection display section
- 12:: Reference display section
- a:: Type-A detection display section
- b:: Type-B detection display section
- c:: Reference display section

### Best Mode for Carrying Out the Invention

The present invention is described in detail below.

The present invention relates to a test device for membrane assay based on measurement principles of a specific binding reaction with a substance to be detected, in which a cationic substance is used for a reference display section positioned on a membrane serving as a membrane carrier. A test device contains a capture reagent capable of specifically binding to a substance to be detected and a labeled reagent capable of specifically binding to a substance to be detected that is labeled with a labeling substance. A substance to be detected forms a "labeled reagent-substance to be detected-capture reagent" sandwich complex with a capture reagent and a labeled reagent provided in a detection display section of a test device. Further, the test device is provided with a reference display section for displaying proper test completion to an operator. An operator can confirm proper test implementation and completion by observing the reference display section. A reference display section may be referred to as a "reaction completion display section" or "validation section." The term "specific binding" used in the present invention indicates selective reactivity between two substances. For example, the term indicates a specific immunological reaction such as an antigen-antibody reaction, a specific reaction such as a reaction between a receptor and its ligand, or specific binding. Examples of such test device described are a flow-through-type test device and a lateral-flow-type test device. In a flow-through-type test device, an analyte passes across the membrane described above. In a lateral-flow-type test device, an analyte is developed and transferred along a membrane. The term "membrane assay" refers to an assay in which at least one step of a reaction is performed on a membrane-type support such that the above specific binding takes place on the support.

Examples of a substance to be detected in the present invention include, but are not limited to: viruses such as influenza virus, adenovirus, RS virus, HAV, HBV, HCV, HIV, EBV, norovirus, rotavirus, and parvovirus, or their components or antibodies; bacteria such as *Escherichia coli,* salmonella, staphylococcus, campylobacter, *Clostridium perfringens, Vibrio parahaemolyticus, Chlamydia trachomatis,* hemolytic streptococcus, *Bordetella pertussis, Helicobacter pylori, Leptospira, Treponema pallidum, Toxoplasma gondii, Borrelia,* anthrax, and MRSA, or their components or antibodies; substances such as mycoplasma lipid, human transferrin, human albumin, human immunogloblin, microglobulin, CRP, troponin, β-glucan, and RF, or their components or antibodies; peptide hormones such as human chorionic gonadotropin or their antibodies; steroids such as steroid hormones or their antibodies; physiologically active amines such as epinephrine and morphine or their antibodies; vitamins such as vitamin B or their antibodies; prostaglandins or their antibodies; antibiotics such as tetracycline or their antibodies; toxins produced by bacteria and the like or their antibodies; a variety of tumor markers or their antibodies; agrichemicals or their antibodies; and polynucleotide or oligonucleotide sequences complementary to pathogenic microorganism-derived nucleic acid sequences.

The term "capture reagent" refers to a reagent that has been immobilized on a membrane so as to bind to any of the above substances to be detected. Such reagent is used for capturing the substance to be detected. If a substance to be detected is an antigen, an antibody against the antigen can be used as a capture reagent. If a substance to be detected is an antibody, an antigen to which the anbibody binds can be used as a capture reagent. In addition, when substances between which a ligand-receptor relationship exists are used, either thereof may be a substance to be detected while the other may be a capture reagent. Further, if a substance to be detected is a nucleic acid, a nucleic acid, which has a sequence complementary to the sequence of the nucleic acid and thus can hybridize to the nucleic acid, can be used as a capture reagent.

According to the present invention, ther term "labeled reagent" refers to a conjugate obtained by allowing an adequate labeling substance to bind to a substance capable of specifically binding to a substance to be detected. If a substance to be detected is an antigen, an antibody against the antigen can be used as a substance capable of specifically binding to a substance to be detected. If a substance to be detected is an antibody, an antigen to which the antibody binds can be used as a substance capable of specifically binding to a substance to be detected. When substances between which a ligand-receptor relationship exists are used, either thereof may be a substance to be detected while the other may be a substance capable of specifically binding to a substance to be detected. Further, if a substance to be detected is a nucleic acid, a nucleic acid, which has a sequence complementary to the sequence of the nucleic acid and thus can hybridize to the nucleic acid, can be used as a substance capable of specifically binding to a substance to be detected. Any labeling substance generally used for labeling of a substance can be used. Examples thereof include: metallic colloidal particles such as gold colloidal particles; nonmetallic colloidal particles such as selenium colloidal particles; particles of a resin substance such as colored latex particles; insoluble particles such as dye colloidal particles and colored liposomes; enzymes that catalyze chromogenic reactions such as alkaline phosphatase and peroxidase; fluorochromes; and radioisotopes. A labeling substance used in the present invention may be a generally available commercial product.

An important feature of the detection device of the present invention is its use of a cationic substance in a reference display section. A reference display section can be provided by immobilizing a cationic substance on a membrane carrier. Here, immobilization of a cationic substance on a membrane carrier can be carried out by allowing a membrane carrier to absorb a cationic substance and then to be dried. It is presumed that a cationic substance retains a strong positive charge so as to cause an ampholyte such as a protein entering the vicinity thereof to be negatively charged, following which it captures the protein via an electrical interaction between the negative charge and the positive charge of the cationic substance itself. This is probably because the pH in the proximity of a strong cationic substance is always basic, which eventually causes an ampholyte such as protein entering the vicinity of a cationic substance to be negatively charged. A cationic substance used in the present invention is strongly cationic to an extent such that it can cause an ampholyte entering the vicinity thereof to be negatively charged. Unlike binding of a labeled reagent caused by a conventional antigen-antibody reaction, a cationic substance has no selectivity (i.e., specificity) in terms of binding subject. A cationic substance can adsorb to any anionic substance or any ampholyte that is negatively charged under basic conditions. That is to say, for the device of the present invention in which a cationic substance is used for a reference display section, a substance capable of specifically binding to a substance to be detected, which is allowed to bind to a labeling substance, is an anionic substance or an ampholyte that is negatively charged under basic conditions. An example thereof is a protein such as an antibody. However, there is concern that binding of a labeled reagent could be inhibited by the binding of a foreign substance in a sample. Such concern can be readily resolved by applying a sufficient amount of a cationic substance. This is a very important feature of the present invention. In general, application of an excessive amount of an antigen, an antibody, or the like to a membrane carrier tends to affect performance in terms of the binding volume or the binding rate. Therefore, the amount of an antibody to be applied cannot be significantly increased. This is probably because an epitope or an epitope recognition site of an antigen or antibody molecule is masked by a different antigen or antibody molecule. The present invention uses electrical interaction and thus the binding volume can be controled by simply increasing or decreasing the amount of the cationic substance to be immobilized. In the present invention polyallylamine is used as a cationic substance to be applied to a reference display section. For example, a cationic polymer to be used is a polymer compound having amino groups or imino groups on the main chain or side chain, a cation charge density of 0.4 meq/g to 21 meq/g (preferably 1.0 meq/g to 21 meq/g and more preferably 4.0 meq/g to 21 meq/g), and an average molecular weight of 300 to 5,000,000. Examples of such cationic polymer described herein include polyethylenimine, and polyvinylamine. Polyethyelenimine includes linear-PEI and branched polyethylenimine comprising primary, secondary, or tertiary amine, and any of them can be used. In addition, the molecular weight of polyethylenimine is not limited. Also, polyethylenimine subjected to chemical modification such as deacylation can be included. Further, examples of polyallylamine include polyallylamine derivatives such as poly(allyl-N-carbamoylguanidino-co-allylamine). Examples of cationic surfactant described herein include benzalkonium chloride and tetradecyl bromide trimethylammonium. A mixture of the above substances may be used. Polyallylamine is desirable in terms of cost, ease of production, and rapid and clear reactivity. The amount of the above cationic substance to be immobilized is not limited. However, the substance is prepared at a concentration of 0.1% to 10% (w/v). Then, 0.1 µl to 10 µl of the substance is added to a membrane of a lateral-flow-type or flow-through-type test device, followed by fixation. In an alternative method, a reference display function can be imparted to such test device by positioning a nylon membrane positively charged via chemical modification on a part of a membrane of a test device such that they overlap each other.

Specific embodiments of the test device of the present invention are described below.

The test device of the present invention is a lateral-flow-type device.

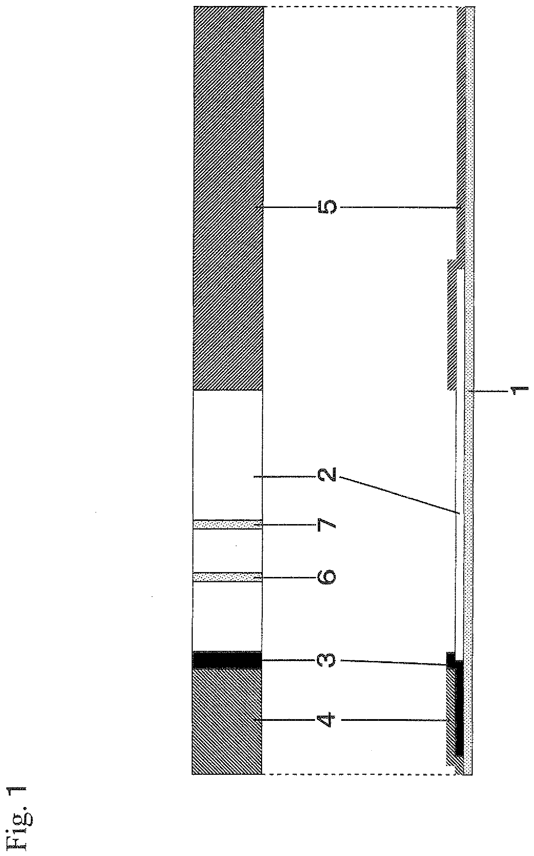

A specific example of the configuration of a lateral-flow-type test device is shown in Fig. 1. The test device shown in Fig. 1 is merely an example and therefore the test device of the present invention is not limited to the configuration shown in Fig. 1 in terms of structure or size. In Fig. 1, numerical references 1, 2, 3, 4, 5, 6, and 7 denote a backing sheet, a membrane carrier, an impregnated member, a sample supply member, a water-absorbing member, a detection display section, and a reference display section, respectively. A backing sheet is a sheet made of plastic or the like and used for maintaining the test device structure. A membrane carrier 2 is composed of a nitrocellulose membrane with a width of 5 mm and a length of 30 mm in a band form. A capture reagent is immobilized at a site 7.5 mm away from the end of a membrane carrier 2 on the side on which chromatgraphic development starts (the left end of a membrane carrier 2 in Fig. 1) such that an analyte detection display section 6 is formed. A known method involving physical adsorption, chemical binding, or the like can be used as a method for fixing a capture reagent on a membrane carrier 2. A capture reagent is immobilized in a linear form on a membrane carrier 2 such that a detection display section 6 is formed. However, it may be immobilized in any form, such as a circular or square form. A cationic substance is immobilized at a site 4.0 mm away from a detection display section 6 on a membrane carrier 2 such that an analyte reference display section 7 is formed. As in the case of a detection display section 6, a known method involving physical adsorption, chemical binding, or the like can be used as a method for fixing a cationic substance on a membrane carrier 2 for a reference display section 7. A cationic substance is immobilized in a linear form on a membrane carrier 2 such that a reference display section 7 is formed. However, it may be immobilized in any form, such as a circular or square form. As shown in the example of Fig. 1, a reference display section is desirably formed downstream of a detection display section on which a capture reagent has been immobilized. In the lateral-flow-type test device of the present invention, the flow of a solution starts from a sample supply member and moves toward a water-absorbing member. Here, the terms "upstream" and "downstream" are defined based on the direction of such flow. A nitrocellulose membrane is described as an example of a membrane carrier 2. However, any membrane can be used as long as it allows chromatographic development of an analyte contained in a test sample, and the capture reagent and the cationic substance described above can be immobilized on it. Examples of a membrane that can be used include other cellulose membranes, nylon membranes, and glass fiber membranes. An impregnated member 3 consists of a member impregnated with a labeled reagent. Nonwoven fabric comprising synthetic fibers in a band form with a size of 5 mm × 8 mm is used for an impregnated member 3. However, the present invention is not limited thereto. For example, cellulose fabric (filter paper, nitrocellulose membrane, or the like) and porous plastic fabric comprising glass fibers, polyethylene, polypropylene, or the like can also be used. A labeled reagent is labeled with a labeling substance and specifically binds to a substance to be detected. Examples of a labeling substance include, but are not limited to, a color labeling substance, an enzyme labeling substance, and a fluorescent labeling substance. Of these, a color labeling substance is preferably used in terms of rapid and convenient determination via visualobservation of color changes in a detection display section 6. Examples of a color labeling substance include latex such as synthetic latex (e.g., polystyrene latex) or natural rubber latex dyed with a colorant such as a red or blue colorant, in addition to a metallic colloid such as gold colloid or platinum colloid. Of these, polystyrene latex is particularly preferable. An impregnated member 3 can be produced by impregnating a member consisting of the above nonwoven fabric comprising synthetic fibers or the like with a suspension of a labeled reagent and drying the impregnated member. As shown in Fig. 1, a membrane carrier 2 is attached to the middle of a backing sheet 1. The downstream end portion of the impregnated member 3 is positioned on or under the end portion of the membrane carrier 2 on the side on which chromatographic development starts (hereinafter referred to as the "upstream side" (i.e., the left side in Fig. 1), while the opposite side thereof is referred to as the "downstream side" (i.e., the right side in Fig. 1)) such that the impregnated member 3 is sequentially connected to the membrane carrier 2. Then, the upstream portion of the impregnated member 3 is attached to the backing sheet 1. Thus, the lateral-flow-type test device of the present invention can be produced. The device in a strip form shown in Fig. 1 may be referred to as a "chromatographic strip." When a substance to be detected is captured using an antigen-antibody reaction, the device is sometimes referred to as an "immunochromatographic device" or "immunochromatographic strip."

Further, if necessary, it is also possible to position the downstream portion of a sample supply member 4 on the upper surface of the impregnated member 3 such that they overlap each other and attach the upstream portion of the sample supply member 4 to the backing sheet 1. It is also possible to position the upstream portion of a water-absorbing member 5 on the upper surface of the downstream portion of the membrane carrier 2 such that they overlap each other and attach the downstream portion of the water-absorbing member 5 to the backing sheet 1. Examples of a sample supply member 4 that can be used include: nonwoven fabric consisting of porous synthetic fibers of porous polyethylene, porous polypropylene, or the like; cellulose paper such as filter paper and cotton fabric; woven fabric; and nonwoven fabric.

The water-absorbing member 5 is a member at which the flow of a liquid sample supplied to the sample supply member 4 arrives. The sample supply member absorbs and retains a flowing sample such that the analyte sample rapidly flows upstream to downstream. Therefore, the water-absorbing member 5 may consist of a material that can rapidly absorb and retain liquid. Examples of such material include cellulose, glass fibers, and nonwoven fabric consisting of porous plastic such as polyethylene or polypropylene. In particular, cellulose is the most suitable material. Further, a lateral-flow-type immunochromatographic device shown in Fig. 1 can be provided with a configuration in which a laminate sheet is attached to the surface of a membrane carrier 2 for protection or in which an opening of a test sample inlet and an opening of a determination window are made on an appropriate plastic housing for a sample supply member 4, a detection display section 6, and a reference display section 7 incorporated into the housing.

When a liquid sample is supplied to a sample supply member 4, the liquid sample permeates the sample supply member 4 and is transferred to an impregnated member 3 by capillary action, and then the liquid sample dissolves a labeled reagent contained in the impregnated member 3. If a substance to be detected is present in a liquid sample, the substance binds to a labeled reagent such that a "labeled reagent-substance to be detected" complex is formed. Such "labeled reagent-substance to be detected" complex further permeates by capillary action and is transferred to a membrane carrier 2 so as to pass through a detection display section 6. At such time, a capture reagent in the detection display section 6 captures the "labeled reagent-substance to be detected" complex such that a "labeled reagent-substance to be detected-capture reagent" sandwich complex is formed. In addition, a portion of the labeled reagent that does not react with a substance to be detected is not captured in the detection display section 6 and is further transferred to a reference display section 7 located downstream of the membrane carrier 2. The portion of a labeled reagent transferred to the reference display section 7 is captured by a cationic substance immobilized on the reference display section 7. In such case, a portion of an anionic substance in a liquid sample might have been bound to the cationic substance on the reference display section 7. However, this is unlikely to influence the capture of the labeled reagent. Then, a portion of the labeled reagent captured by the detection display section 6 and that captured by the reference display section 7 are detected. The most appropriate method for detecting a labeled reagent can be selected depending on a relevant labeling method. For example, in the case of labeling with an enzyme, a substrate is supplied for color development. In the case of labeleing with a fluorescent substance or radioisotope, a dedicated device is used for determination. In the case of labeleing with colored particles such as gold colloidal particles or latex particles, an aggregation image is confirmed by visual observation. When label of the labeled reagent is detected in the reference display section 7, this indicates that the liquid sample supplied to the sample supply member 4 has passed through the detection display section 6 and arrived at the reference display section 7. Accordingly, it can be determined that assay has been normally carried out. If a substance to be detected is present in a liquid sample, labeing of the labeled reagent is detected in the detection display section 6 and in the reference display section 7. If a substance to be detected is not present in a liquid sample, label of the labeled reagent is detected only in the reference display section 7.

In addition, in another embodiment of a lateral-flow-type device, there exist a method wherein a sample supply member 4 is directly positioned on a membrane carrier such that they overlap each other without an impregnated member 3 therebetween, a liquid sample and a labeled reagent are mixed in advance, and the mixture is supplied to a sample supply member 4. When a liquid sample and a labeled reagent are mixed in advance, a substance to be detected in the liquid sample forms a "labeled reagent-substance to be detected" complex. Next, the mixture of a liquid sample and a labeled reagent is supplied to a sample supply member 4. Accordingly, the mixture of a liquid sample and a labeled reagent permeates the sample supply member 4 and is transferred to a membrane carrier by capirally action. The mixture of a liquid sample and a labeled reagent transferred to the membrane carrier passes through a detection display section 6. At such time, a "labeled reagent-substance to be detected" in the mixture of a liquid sample and a labeled reagent is captured by a capture reagent in the detection display section 6 such that a "labeled reagent-substance to be detected-capture reagent" sandwich complex is formed. In addition, a portion of the "labeled reagent-substance to be detected" complex that has not been captured by the capture reagent and a nonreacted portion of the labeled reagent are further transferred to a reference display section 7 located downstream of the detection display section 6 and captured in the reference display section 7.

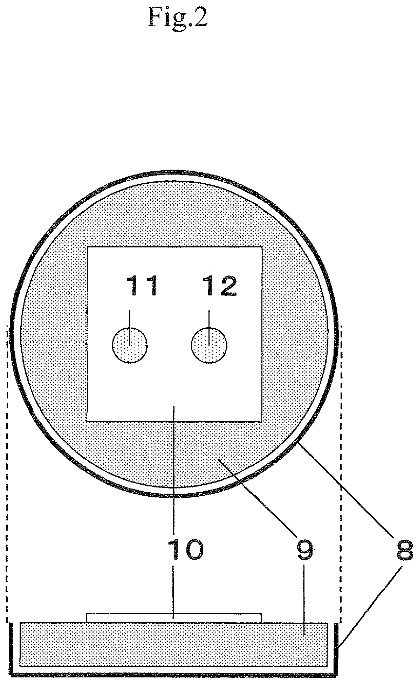

For a flow-through-type device, the configuration shown in Fig. 2 is described as an example. In Fig. 2, numerical references 8, 9, 10, 11, and 12 denote a 35-mm plastic petri dish, a water-absorbing pad, a membrane carrier, a detection display section, and a reference display section, respectively. A nitrocellulose membrane (pore diameter: 3 µm, Toyo Roshi Kaisha, Ltd.) cut into a piece with a size of 20 mm × 20 mm is used for a liquid permeable membrane carrier 10. An analyte detection display section 11 on which a capture reagent has been immobilized is provided on a membrane carrier 10. A known method involving physical adsorption, chemical binding, or the like can be used as a method for fixing a capture reagent on a membrane carrier 10. A capture reagent is immobilized on a membrane carrier 10 in a circular form such that a detection display section 11 is formed. However, it may be immobilized in any form, such as a linear or square form. A reference display section 12 on which a cationic substance has been immobilized is provided to a membrane carrier 10 in a manner such that the reference display seciton 12 and the detection display section 11 exist on the same plane with a certain distance therebetween. As in the case of the detection display section 11, a known method involving physical adsorption, chemical binding, or the like can be used as a method for fixing a reference display section 12 on a membrane carrier 10. A cationic substance is immobilized on a membrane carrier 10 in a circular form such that a reference display section 12 is formed. However, it may be immobilized in any form, such as a linear or square form. A nitrocellulose membrane is used for a membrane carrier 10. However, any membrane can be used as long it allows chromatographic development of an analyte contained in a test sample, and the capture reagent and the cationic substance described above can be immobilized on it. Examples of a membrane that can be used include other cellulose membranes and membranes comprising nonwoven fabric, cotton fibers, plastic fibers such as nylon or urethane fibers, or mixed fibers thereof. A water-absorbing pad 9 with an adequate size (larger than a nitrocellulose membrane with a size of 20 mm × 20 mm) is placed in a plastic petri dish 8. Further, the membrane carrier 10 is placed on the water-absorbing pad 9 such that the face upon which the reagent has been added dropwise and immobilized (upper surface) is observable. A liquid sample is added to the upper surface of the membrane carrier 10. The upper surface of the membrane carrier 10 is referred to as a sample instillation face. A water-absorbing pad 9 is a member at which a flowing supplied liquid sample arrives. It is used as a water-absorbing means for absorbing and retaining a flowing sample. For a water-absorbing means in contact with the lower surface of the membrane carrier, fibers obtained from absorbent cotton or filter paper can be formed into a water-absorbing pad and used as shown in the example of Fig. 2. However, any means, such as a drug comprising a water-absorbing polymer or a vacuum aspirator, may be used as long as it absorbs or aspirates liquid passing through a membrane carrier. In addition, in the example shown in Fig. 2, a plastic petri dish is used. However, the configuration of the test device is not limited to a plastic petri dish. Any configuration may be employed as long as a membrane carrier and a water-absorbing means can be retained by, for example, incorporating them into a plastic device.

When a liquid sample is supplied to the upper surface (sample instillation face) of a membrane carrier 10, the liquid sample passes through the membrane and is absorbed by a water-absorbing pad 9. At such time, if a substance to be detected is present in a liquid sample, the substance to be detected is captured by a capture reagent in a detection display section 11 such that a "substance to be detected-capture reagent" complex is formed. Next, a substance capable of specifically binding to a substance to be detected which is labeled with an enzyme such as peroxidase, a fluorescent substance, a radioisotope, coloring particles such as gold colloidal particles or latex particles, or the like (labeled reagent) is supplied to the reagent instillation face of a membrane carrier 10 such that it is allowed to bind to a substance to be detected that has been captured by a detection display section 11. Thus, a "labeled reagent-substance to be detected-capture reagent" sandwich complex is formed. In addition, a portion of the labeled reagent is captured by a cationic substance immobilized in a reference display section 12. In such case, a portion of the anionic substance in a liquid sample might have been bound to a cationic substance in a reference display section 12. However, this is unlikely to influence the capture of a labeled reagent. Then, the portion of a labeled reagent captured by the detection display section 11 and that captured by the reference display section 12 are detected. The most appropriate method for detecting a labeled reagent can be selected depending on a relevant labeling method. For example, in the case of labeling with an enzyme, a substrate is supplied for color development. In the case of labeleing with a fluorescent substance or radioisotope, a dedicated device is used for determination. In the case of labeling with colored particles such as gold colloidal particles or latex particles, an aggregation image is confirmed by visual observation. If a substance to be detected is present in a liquid sample, label of a labeled reagent is detected in both of the detection display section 11 and the reference display section 12. If a substance to be detected is not present in a liquid sample, label of a labeled reagent is detected only in the reference display section 12.

In addition, another configuration of a flow-through-type device is used for a method wherein a liquid sample and a labeled reagent are mixed in advance and then the mixture is supplied to the sample instillation face of a membrane carrier. When a liquid sample and a labeled reagent are mixed in advance, a substance to be detected in the liquid sample forms a "labeled reagent-substance to be detected" complex. Next, the mixture of a liquid sample and a labeled reagent is supplied to the sample instillation face of a membrane carrier. The mixture of a liquid sample and a labeled reagent passes through a membrane carrier and is absorbed by a water-absorbing means. At such time, a "labeled reagent-substance to be detected" complex in a liquid sample is captured by a captre reagent in a detection display section. Thus, a "labeled reagent-substance to be detected-capture reagent" sandwich complex is formed. In addition, a portion of the "labeled reagent-substance to be detected" complex and a nonreacted portion of the labeled reagent are captured in a reference display section. If label of a labeled reagent is detected in the reference display section, this indicates that a liquid sample supplied to the sample instillation face has passed through the detection display section 11 and the reference display section 12. Accordingly, it can be determined that assay has been normally carried out.

A liquid sample to be tested by the test device of the present invention can be selected from the following examples: clinical liquid specimens such as urine, liquid stool, blood, serum, upper respiratory tract smear, and aspirate fluid; environment-derived aqueous specimens; liquid foods such as drinking water; and solutions and emulsions obtained by disolving or suspensing microorganisms, solid stool, soil, sludge, solid food, atmospheric components, and the like in liquid, and supernatants thereof.

### Examples

The present invention is hereafter described in greater detail with reference to the following examples, although the present invention is not limited thereto.

### Comparative example

### 1: Production of a lateral-flow-type test device

### 1. Production of a latex-labeled reagent

Blue polystyrene latex particles with an average particle diameter of 0.2 µm (Ceradyne, Inc.) were added to an anti-adenovirus monoclonal antibody solution (0.5 mg/mL) previously dialyzed with a 50 mM MES buffer (pH 6.0) to a final concentration of 0.25% (w/v). The resultant was left to stand at 37°C for 2 hours and sometimes subjected to stirring. Next, centrifugation was performed at 5,000 × g at 4°C for 15 minutes so as to remove the supernatant. A Tris buffer (pH 7.5) supplemented with 0.5% (w/v) BSA was added to precipitated latex particles such that the volume of latex particles was adjusted to the initial volume. The latex particles were dispersed again and left to stand at 4°C for 16 hours. Centrifugation was performed again at 5,000 × g at 4°C for 15 minutes so as to remove the supernatant. Precipitated latex particles were dispersed in a Tris buffer (pH 7.5) to a final concentration of 0.1% (w/v). Thus, a latex-labeled reagent was obtained.

### 2. Production of a latex-labeled reagent impregnated member

Glass fiber filter paper (Whatman) was cut into a piece with a size of 5 mm × 10 mm. The latex-labeled reagent (5 µL) produced in 1 above was added dropwise thereto, followed by drying at 37°C for 3 hours. Thus, an impregnated member 3 was obtained.

### 3. Assembly of a lateral-flow-type test device

A lateral-flow-type test device having a configuration similar to the configuration shown in Fig. 1 was used.

As a membrane carrier 2, a nitrocellulose membrane sheet (Millipore) with a size of 5 mm (width) × 30 mm (length) was used. A positive-pressure spray device (BioJet; BioDot) was used to apply a hybridoma-cell-line-derived anti-adenovirus monoclonal antibody solution (5.0 mg/mL) (differing from the solution descriebed in 1 above) serving as a capture reagent to the carrier such that a line was formed 6 mm away from one long-axis end of the carrier (designated as the upstream end, while the opposite end thereof was designated as the downstream end). Further, 1% (w/v) polyethylenimine P-70 (Wako Pure Chemical Industries, Ltd.) serving as a reference reagent was applied to the carrier such that a line was formed 4 mm away from the previously formed line on the downstream side. Thereafter, the carrier was dried under conditions of 37°C for 3 hours. Thus, a detection display section 6 and a reference display section 7 were formed.

Next, the membrane carrier 2 was attached to a plastic backing sheet 1 (BioDot) cut so as to have a width of 5 mm in a manner such that the face opposite from the face to which the reagents had been applied was attached thereto. (In such case, it is necessary for a backing sheet 1 to have an adequate surplus length because it is necessary to separately fix the above impregnated member 3 and the water-absorbing member 5 mentioned below on both ends of the membrane carrier 2.) Subsequently, the impregnated member 3 was attached to the backing sheet 1 on the upstream side of the membrane carrier 2 in a manner such that the impregnated member 3 and the membrane carrier 2 overlapped each other by 1 mm. Then, a filter paper piece (Advantec Toyo Kaisha Ltd.) with a size of 5 mm × 10 mm serving as a sample supply member 4 was attached to the backing sheet in a manner such that the sample supply member 4 and the impregnated member 3 overlapped each other by 9 mm. Further, thick filter paper (Whatman) with a size of 5 mm × 30 mm serving as a water-absorbing member 5 was attached to the backing sheet 1 on the downstream side of the membrane carrier 2 in a manner such that the water-absorbing member 5 and the membrane carrier overlapped each other by 10 mm. In the end, unnecessary portions of the backing sheet which outwardly protruded from both ends were cut off. Thus, the lateral-flow-type test device shown in Fig. 1 was obtained.

### 4. Antigen detection

A 50 mM Tris buffer (pH 8.0) containing 1% (w/v) Triton X-100 and 2% (w/v) arginine hydrochloride was used as a developer, and a model experiment was conducted using an adenovirus CF antigen (Denka Seiken Co., Ltd.) as an analyte. The adenovirus CF antigen was adequately diluted with a developer used as a dilution solvent. Thus, a liquid sample was prepared. The liquid sample (100 µL) was added to a small test tube. The lateral-flow-type test device produced in 3 above was inserted into the test tube such that the sample supply member 4 (on the upstream side) was positioned downward. The test tube was placed upright in a rack and allowed to stand at room temperature. In addition, for a negative control, a similar experiment was conducted with the developer alone (100 µL). At first, the liquid sample was allowed to penetrate the sample supply member 4. Then, it was mixed with a latex-labeled antibody contained in the impregnated member 3 and the mixture permeated the membrane carrier 2 by capillary action. The mixture of the liquid sample and the latex-labeled antibody that had permeated was allowed to pass through the detection display section 6 and the reference display section 7 in such order and was eventually absorbed by the water-absorbing member 5 positioned closest to the downstream end. Thus, the reaction process was completed. After the elapse of time sufficient for line signal formation (20 minutes in this Example), the presence or absence of a blue line appearing in the detection display section 6 and in the reference display section 7 on the nitrocellulose membrane and line characteristics such as width and color tone were observed.

As a result, clear blue lines were observed in the detection display section 6 and in the reference display section 7 of the test device in which the liquid sample had permeated, respectively. It was possible to confirm positive signals in the detection display section 6 with the use of a solution containing the above adenovirus CF antigen diluted up to 1,000 times. Meanwhile, in the case of the test device in which the developer alone (used as a negative control) had permeated, a clear blue line was observed in the reference display section 7, while on the other hand, no line was displayed in the detection display section 6. Accordingly, it was confirmed that a reference display section formed by allowing a membrane carrier to absorb a cationic substance and drying the carrier also can display an intensely colored line and thus sufficiently function as a reference display section.

Here, the test device used in Example 1 is an immunochromatographic strip using an antigen-antibody reaction. Such immunochromatographic strip can be incorporated into a plastic housing and the resulting product can be used as an immunochromatographic device.

### Comparative example

### 2: Production of a flow-through-type test device

### 1. Production of a gold-colloid-labeled antibody

An anti-A-type or anti-B-type influenza virus NP monoclonal antibody (100 µg/mL) (1 volume) previously dialyzed with a 2 mM sodium borate aqueous solution was added to a gold colloid solution (BBI; particle size: 40 nm; chlorauric acid concentration: 0.01% (w/v)) (9 volumes) adjusted to pH 5.5 with a potassium carbonate aqueous solution (final concentration: 10 µg/mL), followed by slow stirring for 5 minutes. Thereafter, a 10% (w/v) BSA solution (1 volume) was added thereto, followed by further stirring for 10 minutes. Subsequently, the total volume of the resultant was centrifuged at 14,000 rpm at 4°C for 30 minutes so as to remove the supernatant. 1% (w/v) BSA and a 10 mM Tris buffer (pH 8.5) supplemented with 150 mM sodium chloride were added to precipitated gold colloidal particles for redispersion. The dispersion was diluted such that the absorbance at 530 nm became 2.0. In the end, equal amounts of a gold-colloid-labeled anti-A type antibody and a gold-colloid-labeled anti-B type antibody produced in the manner described above were mixed together such that a gold-colloid-labeled anti-influenza antibody was obtained.

### 2. Application of a reagent to a nitrocellulose membrane

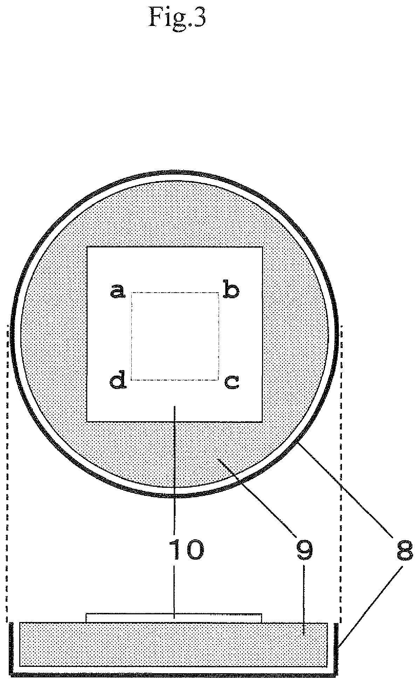

A flow-through-type test device having a configuration similar to the configuration shown in Fig. 3 was used.

A nitrocellulose membrane (pore diameter: 3 µm; Toyo Roshi Kaisha, Ltd.) was cut into a piece with a size of 20 mm × 20 mm. The piece was used as a liquid permeable membrane carrier 10. Supposing that a square with a size of 10 mm × 10 mm was drawn at the center of such a nitrocellulose membrane, the points that would correspond to the four corners of such a square were denoted by "a," "b," "c," and "d" in a clockwise direction starting from one arbitrary corner.

The anti-A-type and anti-B-type influenza virus NP monoclonal antibodies, each of which had been obtained from an hybridoma cell line other than that of the antibody described in 1 above, were separately dialyzed in advance with a 10 mM phosphate buffer (pH 7.0) so as to adjust the concentration to 3 mg/mL.

An anti-A-type influenza virus NP monoclonal antibody and an anti-B-type influenza virus NP monoclonal antibody were added dropwise in an amount of 1 µL to points "a" and "b" on the nitrocellulose membrane, respectively. Thus, a type-A detection display section "a" and a type-B detection display section "b" were formed. Subsequently, 1% polyethylenimine P-70 (Wako Pure Chemical Industries, Ltd.) (1 µL) was added dropwise to point "c" such that a reference display section "c" was formed. No antibody was added to the remaining point "d," and point "d" was designated as a blank and used as a mark to discern the locations of points "a," "b," and "c" after the completion of the test. (Alternatively, it is possible to make a small mark at point "d" with a pencil or the like.)

The nitrocellulose membrane onto which the reagents had been added dropwise was dried at 37°C for 3 hours.

### 3. Assembly of a flow-through-type test device

A cellulose water-absorbing pad 9 (Vessel Inc.) with a size greater than the size of the above membrane carrier 10 (20 mm × 20 mm) was placed in a plastic petri dish 8 with a diameter of 35 mm. Further, the membrane carrier 10 was positioned on the water-absorbing pad 9 such that the face onto which the reagent had been added dropwise (upper surface) was observable. Thus, a flow-through-type immunochromatographic device was obtained.

### 4. Antigen detection

A 10 mM phosphate buffer (pH 7.4) containing 5% (w/v) BSA, 5% (w/v) Triton X-100, and 2% (w/v) gelatin was used as an analyte diluent. An A-type influenza virus or a B-type influenza virus, which had been adequately diluted, was used as a positive analyte. The above analyte diluent was used as a negative analyte.

The gold-colloid-labeled anti-influenza antibody obtained in 1 above was mixed with each analyte diluted with the above analyte dilutent at a ratio of 1:4. Each resultant (1 mL) was added dropwise onto a nitrocellulose membrane of the test device obtained in 3 above. Dropwise addition was performed slowly and gradually and the membrane carrier 10 was left to stand at room temperature until no liquid was observed thereon. Then, the presence or absence of red spots derived from gold colloid was determined.

As a result, the type-A influenza virus was detected at up to 10⁵ pfu/mL in the A-type detection display section "a," and this section gave negative results for the B type influenza virus. The B type influenza virus was detected at up to 10⁵ pfu/mL in the B-type detection display section "b," and this section gave negative results for the A type influenza virus. Meanwhile, in the case of the reference display section "c," an intensely coloerd spot was formed in any case regardless of the analyte type or concentration, and thus the section was confirmed to sufficiently function as a reference display section.

### Example 3: Comparison and examination of reference display reagents 1. Production of lateral-flow-type test devices

Lateral-flow-type test devices were produced using the following 7 types of reagents as candidate reagents used for reference display sections in the manner described in Example 1.

(1) 3.0 mg/mL rabbit anti-mouse immunoglobulin antibody (Dako): A conventional method
(2) 1% (w/v) polyethylenimine P-70 (Wako Pure Chemical Industries, Ltd.): A cationic substance
(3) 1% (w/v) polyallylamine HCl-10S (Nitto Boseki Co., Ltd.): A cationic substance
(4) 1% (w/v) carboxymethyl cellulose (Wako Pure Chemical Industries, Ltd.): An anionic substance
(5) 1% (w/v) sericin (Toyo Boseki Kabushiki Kaisha): An anionic substance
(6) 1% (w/v) polyethylene glycol (Nacalai Tesque, Inc.): A nonionic substance
(7) 1% (w/v) polyvinylalcohol (Nacalai Tesque, Inc.): A nonionic substance

### 2. Test method

As in Example 1, a 50 mM Tris buffer (pH 8.0) containing 1% (w/v) Triton X-100 and 2% (w/v) arginine hydrochloride was used as a developer and an adenovirus CF antigen (Denka Seiken Co., Ltd.) was used as an analyte. A developer was used as a dilution solvent for adequate dilution of an adenovirus CF antigen. The analyte diluted 100 times was desigated as a strongly positive analyte. The analyte diluted 500 times was designated as a weakly positive analyte. Thus, liquid samples were prepared. A test was conducted in the manner described in Example 1. The presence or absence of a blue line appearing in the reference display section 7 of each test device and line characteristics such as width and color were observed.

### 3. Results and discussion

The test devices for which different reagents had been used in the respective reference display sections 7 were examined based on comparisons related to formation of a line in a reference display section 7, the duration for the appearnce of such a line, and influence of such a line upon a line appearing in a detection display section 6 (table 1). As a result of a comparison of the line observed in the case of a conventional method of (1) with other lines, particularly in the cases of (2) and (3), the cationic-subsance-derived line exhibited a darker and clearer color tone than that observed in the case of a conventional method of (1). In addition, the duration of the appearance of the line was shorter than that in the case of (1). In the other cases involving the use of reference reagents each comprising an anionic substance or a nonionic substance, no line was formed, and the relevant reference display sections 7 did not properly function. Sericin used in (5) is a protein comprising cationic amino acid residues. However, the protein molecule as a whole is negatively charged. Thus, it is thought that sericin did not function as a reference display reagent in the above case. In addition, when any of the above reference display reagents was used, no influence on the corresponding detection line was confirmed, regardless of the antigen concentration in the relevant sample. Probably, this is because a reference display section 7 was located downstream of a detection display section 6.

**[Table 1]**

| Reference display reagent | Detection line intensity (strongly positive analyte) | Detection line intensity (weakly positive analyte) | Reference line intensity | Time of appearance of reference line (mean value of 3 measruement results) | Reference line characteristics |
|---|---|---|---|---|---|
| (1) | +++ | + | ++ | 25 (sec.) | |
| (2) | +++ | + | +++ | 20 | Thin line with dark color tone |
| (3) | +++ | + | +++ | 15 | Thick line with dark color tone |
| (4) | +++ | + | - | - | |
| (5) | +++ | + | - | - | |
| (6) | +++ | + | - | - | |
| (7) | +++ | + | - | - | |

| | | | | | |
|---|---|---|---|---|---|
| -: Negative +: Weakly positive (detectable as positive even with light color tone and lack of line clarity) ++: Positive (clearly positive) +++: Strongly positive (clearly positive with particularly dark color tone) | | | | | |

## Claims

1. A test device for membrane assay using a specific binding reaction of a substance to be detected with a capture reagent immobilized on a membrane carrier and a reagent labeled with a labeling substance, the reagent being an anionic substance or an ampholyte that is negatively charged under basic conditions, which comprises a reference display section for indicating proper test completion on which a cationic polymer for capturing a labeled reagent has been immobilized,
wherein the cationic polymer is a polymer compound having a cation charge density of 0.4 meq/g to 21 meq/g, and an average molecular weight of 300 to 5,000,000,
wherein the cationic polymer is polyallylamine,
wherein the test device is a lateral-flow-type test device,
and wherein the reference display section for indicating proper test completion is configured such that it is positioned downstream of a detection display section on which a capture reagent has been immobilized.

2. The test device according to claim 1, wherein the reference display section is configured by allowing the membrane carrier to absorb the cationic polymer and drying the membrane.

3. The test device according to claim 1 or 2, wherein the specific binding reaction is an antigen-antibody reaction.

4. A method for confirming the proper completion of a test for membrane assay using the test device according to any one of claims 1 to 3, the method using a specific binding reaction of a substance to be detected with the capture reagent immobilized on the membrane carrier and the reagent labeled with the labeling substance, whereby proper test completion can be confirmed when labeling of the labeled reagent is detected in the reference display section on the membrane by allowing the labeled reagent to bind to the cationic polymer immobilized in the reference display section.

## Patentansprüche

1. Testvorrichtung für Membranassay unter Verwendung einer spezifischen Bindungsreaktion einer zu detektierenden Substanz mit einem auf einem Membranträger immobilisierten Einfangreagens und eines mit einer Markierungssubstanz markierten Reagens, wobei das Reagens eine anionische Substanz oder ein Ampholyt ist, die/der unter basischen Bedingungen negativ geladen ist, umfassend einen Referenz-Anzeigeabschnitt zum Anzeigen des korrekten Abschlusses des Tests, auf dem ein kationisches Polymer zum Einfangen eines Markierungsreagens immobilisiert wurde,
wobei das kationische Polymer eine Polymerverbindung ist, die eine Kationenladungsdichte von 0,4 meq/g bis 21 meq/g und ein durchschnittliches Molekulargewicht von 300 bis 5.000.000 hat,
wobei das kationische Polymer Polyallylamin ist,
wobei die Testvorrichtung eine Testvorrichtung des Lateralflusstyps ist,
und wobei der Referenz-Anzeigeabschnitt zum Anzeigen zum Anzeigen des korrekten Abschlusses des Tests so konfiguriert ist, dass er stromabwärts von einem Detektionsanzeigeabschnitt, auf dem ein Einfangreagens immobilisiert wurde, positioniert ist.

2. Testvorrichtung gemäß Anspruch 1, wobei der Referenz-Anzeigeabschnitt konfiguriert wird durch Erlauben, dass der Membranträger das kationische Polymer absorbiert, und Trocknen der Membran.

3. Testvorrichtung gemäß Anspruch 1 oder 2, wobei die spezifische Bindungsreaktion eine Antigen-Antikörper-Reaktion ist.

4. Verfahren zum Bestätigen des korrekten Abschlusses eines Tests für Membranassay unter Verwendung der Testvorrichtung gemäß mindestens einem der Ansprüche 1 bis 3, wobei das Verfahren eine spezifische Bindungsreaktion einer zu detektierenden Substanz mit dem auf dem Membranträger immobilisierten Einfangreagens und das mit der Markierungssubstanz markierte Reagens verwendet, wobei der korrekte Abschluss des Tests bestätigt werden kann, wenn die Markierung des markierten Reagens in dem Referenz-Anzeigeabschnitt auf der Membran detektiert wird, durch Erlauben, dass das markierte Reagens an das kationische Polymer, das in dem Referenz-Anzeigeabschnitt immobilisiert ist, bindet.

## Revendications

1. Appareil d'analyse pour essai sur membrane utilisant une réaction de liaison spécifique d'une substance à détecter avec un réactif de capture immobilisé sur un support de membrane et un réactif marqué avec une substance de marquage, le réactif étant une substance anionique ou un ampholyte qui est chargé négativement en condition basique, qui comprend une section d'affichage de référence servant à indiquer l'achèvement correct de l'analyse sur laquelle un polymère cationique servant à capturer un réactif marqué a été immobilisé,
dans lequel le polymère cationique est un composé polymère présentant une densité de charge cationique de 0,4 meq/g à 21 meq/g, et une masse moléculaire moyenne de 300 à 5 000 000,
dans lequel le polymère cationique est une polyallylamine,
dans lequel l'appareil d'analyse est un appareil d'analyse à écoulement latéral ;
et dans lequel la section d'affichage de référence servant à indiquer l'achèvement correct de l'analyse est configurée de telle sorte qu'elle est positionnée en aval d'une section d'affichage de détection sur laquelle un réactif de capture a été immobilisé.

2. Appareil d'analyse selon la revendication 1, dans lequel la section d'affichage de référence est configurée en permettant au support de membrane d'absorber le polymère cationique et en séchant la membrane.

3. Appareil d'analyse selon la revendication 1 ou 2, dans lequel la réaction de liaison spécifique est une réaction antigène-anticorps.

4. Procédé de confirmation de l'achèvement correct d'une analyse pour un essai sur membrane utilisant l'appareil d'analyse selon l'une quelconque des revendications 1 à 3, le procédé utilisant une réaction de liaison spécifique d'une substance à détecter avec le réactif de capture immobilisé sur le support de membrane et le réactif marqué avec la substance de marquage, moyennant quoi l'achèvement correct de l'analyse peut être confirmé quand le marquage du réactif marqué est détecté dans la section d'affichage de référence sur la membrane en permettant au réactif marqué de se lier au polymère cationique immobilisé dans la section d'affichage de référence.
